# EUROPÄISCHE PATENTSCHRIFT

(11) **EP 2 268 715 B1**
(45) Veröffentlichungstag und Bekanntmachung des Hinweises auf die Patenterteilung: **15.07.2015**
(21) Anmeldenummer: 09731782.0
(22) Anmeldetag: 15.04.2009
(51) Int. Cl.: C08J 3/00, C08J 3/28, A61L 27/22, A61L 27/52, C08J 7/12, A23L 1/0562, A61L 15/60, C08J 9/00

(54) **VERFAHREN ZUR HERSTELLUNG EINES SCHNELL BENETZBAREN FORMKÖRPERS**
METHOD FOR THE MANUFACTURE OF A RAPIDLY WETTABLE MOULDING
PROCÉDÉ DE PRODUCTION D'UN CORPS MOULÉ RAPIDEMENT MOUILLABLE

(30) Priorität: 15.04.2008 DE 102008020197
(43) Veröffentlichungstag der Anmeldung: 05.01.2011
(73) Patentinhaber: GELITA AG, 69412 Eberbach (DE)
(72) Erfinder: AHLERS, Michael, 69412 Eberbach (DE); ÖSSER, Steffen, 24960 Glücksburg (DE); GRZINIA, Michael, 71403 Schwaikheim (DE); FLECHSENHAR, Klaus, 69412 Eberbach (DE)
(74) Vertreter: Hoeger, Stellrecht & Partner Patentanwälte
(86) Internationale Anmeldenummer: PCT/EP2009/054452
(87) Internationale Veröffentlichungsnummer: WO 2009/127653

(56) Entgegenhaltungen:
- EP-A- 0 122 289
- WO-A-2005/067995
- WO-A-2005/111121
- WO-A-2008/018009
- WO-A-2008/093342
- WO-A-2009/052998
- DE-A1- 10 037 048
- DE-A1-102004 027 802
- JP-A- 3 290 446
- US-A1- 2003 232 071
- MARKOWICZ M ET AL: "The impact of vacuum freeze-drying on collagen sponges after gas plasma sterilization" 1. Januar 2006 (2006-01-01), JOURNAL OF BIOMATERIALS SCIENCE. POLYMER EDITION, VSP, UTRECHT, PAGE(S) 61 - 75 , XP009109772 ISSN: 0920-5063 Zusammenfassung Seite 62, Absatz 1 - Seite 74, letzter Absatz
- KOSE G T ET AL: "Tissue engineered cartilage on collagen and PHBV matrices" 1. September 2005 (2005-09-01), BIOMATERIALS, ELSEVIER SCIENCE PUBLISHERS BV., BARKING, GB, PAGE(S) 5187 - 5197 , XP025280837 ISSN: 0142-9612 [gefunden am 2005-09-01] Zusammenfassung Seite 5188, rechte Spalte, Absatz 2 - Seite 5196, rechte Spalte, Absatz 2
- DATABASE WPI Week 199228 Thomson Scientific, London, GB; AN 1992-229946 XP002536258 -& JP 04 153381 A (KANEBO LTD) 26. Mai 1992 (1992-05-26)
- DATABASE WPI Week 200065 Thomson Scientific, London, GB; AN 2000-670091 XP002536259 -& JP 2000 279804 A (TOPPAN PRINTING CO LTD) 10. Oktober 2000 (2000-10-10)
- LIEDERMANN K ET AL: "Dielectric spectroscopy of plasma modified polysaccharides for medical applications" PROPERTIES AND APPLICATIONS OF DIELECTRIC MATERIALS, 1997., PROCEEDING S OF THE 5TH INTERNATIONAL CONFERENCE ON SEOUL, SOUTH KOREA 25-30 MAY 1997, NEW YORK, NY, USA,IEEE, US, Bd. 1, 25. Mai 1997 (1997-05-25), Seiten 541-544, XP010242511 ISBN: 978-0-7803-2651-4

## Beschreibung

Gegenstand der vorliegenden Erfindung ist ein Verfahren zur Herstellung eines schnell benetzbaren Formkörpers aus einem natürliches Hydrokolloid enthaltenden Material.

Als Hydrokolloide werden eine Reihe verschiedener polymerer Stoffe bezeichnet, die meist natürlichen Ursprungs sind (insbesondere Polysaccharide und Proteine), zum Teil aber auch synthetisch hergestellt werden. Sie zeichnen sich durch die Eigenschaft aus, in wässrigen Systemen Gele oder viskose Lösungen auszubilden, wobei einige Hydrokolloide (in Abhängigkeit von der Temperatur) wasserlöslich sind, andere hingegen in Form von kolloiden Lösungen, Dispersionen oder Emulsionen vorliegen. Diese und andere Eigenschaften der Hydrokolloide können in einigen Fällen über den Hydrolysegrad beeinflusst werden, beispielsweise bei der Herstellung von löslicher Gelatine aus unlöslichem Kollagen.

Aufgrund dieser Eigenschaften sowie ihrer physiologischen Unbedenklichkeit finden natürliche Hydrokolloide eine breite Anwendung bei der Herstellung von Lebensmittel und Kosmetika als Verdickungsmittel oder Stabilisatoren, d.h. um eine bestimmte Konsistenz oder Textur der Produkte einzustellen. Darüber hinaus werden Hydrokolloide oder Hydrokolloid enthaltende Materialien auch in der Medizin eingesetzt, z.B. bei der Wundversorgung oder als Substrat für lebende Zellen, wobei auch hier die gute physiologische Verträglichkeit, die biologische Abbaubarkeit und die Gelbildungseigenschaften vieler Hydrokolloide vorteilhaft ausgenutzt werden können.

Ein Problem, das bei der Verwendung von Hydrokolloiden in allen oben genannten Bereichen häufig auftritt, ist deren schlechte bzw. geringe Benetzbarkeit. Dieses Phänomen ist vermutlich dadurch bedingt, dass im Zuge der Herstellung bzw. Gewinnung der Hydrokolloide in der Regel eine Trocknung durchgeführt werden muss, wobei sich im Kontakt mit der Luft hydrophobe Bereiche der an sich hydrophilen Hydrokolloidmoleküle zur Luft hin orientieren, sodass die gebildeten Partikel, Agglomerate oder Formkörper eine wasserabweisende Oberfläche aufweisen.

Diese schlechte Benetzbarkeit hat zur Folge, dass z.B. im Lebensmittelbereich zum Lösen bzw. Dispergieren eines Hydrokolloidpulvers eine längere Zeit, starke Agitation oder eine höhere Temperatur erforderlich sind, als dies bei einem schnell benetzbaren Produkt der Fall wäre. Im industriellen Bereich führt dies zu höheren Kosten, während es beim Endverbraucher die Handhabbarkeit des Produkts (z.B. Gelatinepulver) verringert.

Insbesondere für verschiedene medizinische Anwendungen werden Hydrokolloide in Gestalt von Formkörpern eingesetzt. Auch hier sind grundsätzlich solche Hydrokolloid enthaltende Materialien wünschenswert, die beim Kontakt mit wässrigen Medien, insbesondere Körperflüssigkeiten, schnell benetzbar sind.

Die US 2003/0232071 A1 offenbart ein biomimetisches Composite als Knochenersatzmaterial. Dieses Composite umfasst eine organische, mit Flüssigkeit quellbare Matrix und eine anorganische Mineralphase, wobei die quellbare Matrix z.B. Kollagen umfassen kann. Weiterhin wird eine Oberflächenmodifikation der quellbaren Matrix u.a. durch eine Plasmabehandlung offenbart.

In dem Artikel von M. Markowicz et al. in J. Biomater. Sei. Polymer Edn. 17 (2006) 61-75 wird ein Verfahren zur Sterilisation von porösen Kollagenschwämmen unter Anwendung eines Gasplasmas offenbart.

Der Artikel von G. Köse et al. in Biomaterials 26 (2005) 5187-5197 offenbart Kollagenschwämme zur Besiedlung mit Chondrocyten, welche durch Anwendung von Gammastrahlung sterilisiert werden.

Zur Lösung dieses Problems wird erfindungsgemäß ein Verfahren der Eingangs genannten Art vorgeschlagen, beim dem ein Gelatine enthaltendes Material in Gestalt eines Formkörpers einem Plasma ausgesetzt wird.

Es wurde überraschender Weise festgestellt, dass durch die Behandlung eines Hydrokolloid enthaltenden Materials mit einem Plasma, d.h. einem zumindest teilweise ionisierten Gas, dessen Benetzbarkeit ganz deutlich verbessert werden kann, sodass das resultierende Material häufig in kürzester Zeit vollständig benetzbar ist. Gleichzeitig bleiben jedoch die vorteilhaften Eigenschaften der Hydrokolloide im Wesentlichen unbeeinflusst, insbesondere bleibt auch der pH-Wert an der Oberfläche des Hydrokolloid enthaltenden Materials im Wesentlichen unverändert. Dies deutet darauf hin, dass trotz der extremen Bedingungen, die ein Plasma darstellt, offenbar nur in geringem Umfang eine

Die Erfindung betrifft ein Verfahren zur Herstellung eines schnell benetzbaren Formkörpers aus einem Gelatine enthaltenden Material, bei dem ein Formkörper aus einem Gelatine enthaltenden Material mit einer Zellstruktur zuerst mechanisch komprimiert und anschließend einem Plasma ausgesetzt wird. chemische Modifikation des Hydrokolloids erfolgt, welche aber dennoch ausreicht, um die Benetzbarkeit deutlich zu verbessern.

### Hydrokolloid enthaltende Materialien

Für das erfindungsgemäße Verfahren ist das Hydrokolloid ausgewählt aus Gelatine.

Vorzugsweise besteht das Hydrokolloid enthaltende Material zu überwiegenden Teilen aus Hydrokolloid, d.h. zu mehr als 50 Gew.%. Auch wenn weitere, gegebenenfalls wasserlösliche Komponenten in dem Material enthalten sind, kann sich eine mangelhafte Benetzbarkeit aufgrund des Hydrokolloidanteils ergeben. Weitere Komponenten, die in dem Hydrokolloid enthaltenden Material bevorzugt enthalten sein können, werden weiter unten im Zusammenhang mit besonderen Ausführungsformen der Erfindung im Detail aufgeführt.

Bei bevorzugten Ausführungsformen der Erfindung umfasst das Material mehr als ca. 80 Gew.%, vorzugsweise mehr als ca. 90 Gew.% Hydrokolloid. Insbesondere kann das Material auch im Wesentlichen vollständig aus Hydrokolloid bestehen.

Bei der Zusammensetzung des Materials ist zu berücksichtigen, dass das Hydrokolloid einen bestimmten Anteil an Wasser enthalten kann, welches im Gleichgewicht mit der Umgebungsluft von dem Hydrokolloid gebunden wird. Das Hydrokolloid enthaltende Material kann in dieser Form für das erfindungsgemäße Verfahren eingesetzt werden. Alternativ kann der Wassergehalt des Hydrokolloids vor der Plasmabehandlung durch Trocknen reduziert werden, wodurch in einigen Fällen eine erhöhte Flüssigkeitsaufnahmekapazität des hergestellten Materials erzielt werden kann.

Das Hydrokolloid enthaltende Material kann zur Durchführung des erfindungsgemäßen Verfahrens in Form eines Pulvers, Granulats oder Agglomerats vorliegen. Hydrokolloide in solcher Form kommen insbesondere im Lebensmittel- und Kosmetikbereich als Rohstoffe zum Einsatz. Durch das erfindungsgemäße Verfahren können entsprechende Pulver, Granulate oder Agglomerate hergestellt werden, die schnell benetzbar sind und somit schneller und kostengünstiger verarbeitet werden können.

Das Hydrokolloid enthaltende Material liegt gemäß der Erfindung als Formkörper vor. Ein Formkörper im Sinne der vorliegenden Erfindung weist eine vorgegebene räumliche Struktur auf, im Gegensatz etwas zu einem Pulver. Die Formkörper können massiv oder hohl sein oder eine Zellstruktur aufweisen. Einige Beispiele für Formkörper umfassen Folien, Schwämme, Gewebe, Vliese, Röhrchen, Partikel oder Kugel. Bevorzugte Ausführungsformen solcher Formkörper und deren Verwendung werden weiter unten im Detail beschrieben.

### Plasmabehandlung

Das Plasma ist bei dem erfindungsgemäßen Verfahren bevorzugt ein Niederdruckplasma, d.h. ein Plasma, dessen Druck deutlich unter dem Normaldruck von 1013,25 mbar liegt. Insbesondere kann das Verfahren mit einem Niederdruckplasma von ca. 1 mbar oder weniger, bevorzugt ca. 0,5 mbar oder weniger, weiter bevorzugt ca. 0,2 mbar oder weniger durchgeführt werden.

Vorteilhafterweise ist das Plasma ein O₂-Plasma und/oder ein H₂O-Plasma. Die Erzeugung entsprechender Plasmen ist aus dem Stand der Technik bekannt. Das Verfahren wird bevorzugt in der Weise durchgeführt, dass das Hydrokolloid enthaltende Material in einem geschlossenen Raum, in welchem das Plasma erzeugt wird, diesem ausgesetzt wird. Das erfindungsgemäße Verfahren kann insbesondere in einer kommerziell erhältlichen Plasmaanlage durchgeführt werden.

Die Zeitdauer, für die das Hydrokolloid enthaltende Material dem Plasma ausgesetzt wird, kann je nach Art des Hydrokolloids sowie Gestalt und Größe des Materials variiert werden, um den gewünschten Effekt auf die Benetzbarkeit zu erzielen. In den meisten Fällen sollte das Material dem Plasma länger als ca. 1 Minute ausgesetzt werden, um einen merklichen Effekt zu erreichen. Bevorzugt beträgt die Zeitdauer ca. 5 bis ca. 180 Minuten, weiter bevorzugt ca. 20 bis ca. 60 Minuten.

Das erfindungsgemäße Verfahren wird so durchgeführt, dass mindestens ein Teil der Oberfläche des Hydrokolloid enthaltenden Materials dem Plasma ausgesetzt wird. Bevorzugt wird jedoch die gesamte Oberfläche dem Plasma ausgesetzt.

### Gelatine enthaltende Materialien

Bei einer bevorzugten Ausführungsform der Erfindung umfasst das Hydrokolloid Gelatine. Gelatine spielt unter den Hydrokolloiden nicht nur bei der Herstellung von Lebensmitteln eine herausragende Rolle, sondern eignet sich auch in besonderer Weise als Ausgangsprodukt für verschiedene Materialien für die medizinische Anwendung.

Gerade im Hinblick auf die medizinische Anwendung zeichnet sich Gelatine bei entsprechender Reinheit durch eine sehr gute Gewebe- und Zellverträglichkeit und eine im Wesentlichen vollständige Bioabbaubarkeit aus. Diese Eigenschaften bleiben durch die erfindungsgemäße Plasmabehandlung im Wesentlichen unbeeinflusst. Insbesondere ist auch das plasmabehandelte Material sehr gut zellverträglich und es erfolgt keine messbare Änderung des pH-Wertes an der Oberfläche, was z.B. bei einer Verwendung von Gelatine enthaltenden Materialien als Substrat für lebende Zellen von Bedeutung ist.

Die für das erfindungsgemäße Verfahren eingesetzte Gelatine kann chemisch modifiziert sein. Die Modifikation kann insbesondere darin bestehen, dass einzelne Aminosäureseitenketten mit zusätzlichen funktionellen Gruppen versehen werden, um die Affinität des Gelatine enthaltenden Materials gegenüber bestimmten Gewebe- oder Zelltypen zu erhöhen oder zu reduzieren.

Gemäß einer weiteren Ausführungsform der Erfindung ist die Gelatine zumindest partiell vernetzt. Durch eine Vernetzung der Gelatine, die chemisch und/oder enzymatisch erfolgen kann, wird diese in eine unlösliche Form überführt, sodass sich die entsprechenden, vernetzte Gelatine enthaltenden Materialien bei Kontakt mit einem wässrigen Medium bei erhöhter Temperatur (z.B. 37 °C) nicht sofort auflösen, sondern erst im Verlauf einer bestimmten Zeit hydrolytisch abgebaut werden. Die Länge dieser Abbauzeit kann über den Vernetzungsgrad variiert werden.

Eine solche Vernetzung zur Verringerung der Löslichkeit des Materials kann in analoger Weise auch bei anderen wasserlöslichen Hydrokolloiden durchgeführt werden.

Interessanterweise wurde festgestellt, dass durch die erfindungsgemäße Plasmabehandlung zwar die Benetzbarkeit des Hydrokolloids verbessert wird, die Löslichkeit des Materials und das Abbauverhalten des daraus gebildeten Formkörpers jedoch nicht oder nicht wesentlich beeinflusst werden.

### Gelatineschwämme

Ein besonderer Aspekt der vorliegenden Erfindung betrifft die Anwendung des Verfahrens bei einem Gelatine enthaltenden Material, welches eine Zellstruktur aufweist. Solche Materialien mit Zellstruktur werden im Folgenden als Schwämme bezeichnet.

Gelatineschwämme kommen in der Medizin insbesondere zur Förderung der Hämostase, d.h. zum Stillen von Blutungen (z.B. als Wundauflage oder bei Operationen) oder als Substrat für lebende Zellen (z.B. bei der Herstellung von Gewebeimplantaten) zum Einsatz. In allen diesen Fällen ist eine vollständige Benetzung und Hydratisierung des Schwammes vor oder bei der Anwendung erwünscht, sodass schnell benetzbare Gelatineschwämme, die mittels des erfindungsgemäßen Verfahrens hergestellt sind, wesentlich effektiver eingesetzt werden können.

Die aus dem Stand der Technik bekannten Gelatineschwämme müssen aufgrund ihrer schlechten Benetzbarkeit zuvor durch Eintauchen in Wasser angefeuchtet werden, bevor sie z.B. als Wundauflage eingesetzt werden können, da sie nur in feuchtem Zustand eine merkliche Absorptionsfähigkeit aufweisen. Demgegenüber weisen Gelatineschwämme, die nach dem erfindungsgemäßen Verfahren hergestellt wurden, bereits in trockenem Zustand eine erstaunlich hohe Absorptionsfähigkeit gegenüber Blut auf. Auch die Absorptionskapazität ist im Vergleich zu nicht-plasmabehandelten Gelatineschwämmen deutlich höher, d.h. es wird insgesamt eine größere Menge an Blut im Laufe der Zeit aufgenommen.

Gelatineschwämme als solche können z.B. durch das in der DE 10 2004 024 635 A1 beschriebene Verfahren hergestellt werden, bei dem eine wässrige Gelatinelösung aufgeschäumt und anschließend getrocknet wird. Eine Vernetzung der Gelatine kann dabei durch Zugabe eines Vernetzungsmittels in die Gelatinelösung und/oder durch Einwirkung eines Vernetzungsmittels auf den hergestellten Schwamm erfolgen. Der Gelatineschwamm kann daneben weitere Komponenten wie z.B. Weichmacher enthalten.

Die Gelatineschwämme können in Abhängigkeit vom gewünschten Applikationsort in verschiedener Gestalt vorliegen. Bevorzugte Formen sind z.B. Quader mit verschiedenen Abmessungen als Wundauflage oder für Operationen, Zylinder zur Verwendung als Tampons, Schwammfolien zur Versorgung großflächiger Wunden oder Schwämme in Form von Kugeln.

Die Zellstruktur der Gelatineschwämme weist bevorzugt einen mittleren Porendurchmesser von weniger als ca. 300 µm auf. Porendurchmesser in diesem Bereich sind für die oben genannten Anwendungen gut geeignet, insbesondere auch für eine Besiedlung des Schwammes mit lebenden Zellen.

Das erfindungsgemäße Verfahren umfasst ein mechanisches Komprimieren des Gelatine enthaltenden Materials. Durch dieses Komprimieren eines in trockenem Zustand spröden Gelatineschwamms wird ein teilweises Aufbrechen der Zellstruktur bewirkt und ein offenporiges Material erhalten. Dieses Aufbrechen der Zellstruktur erfolgt, ohne dass der strukturelle Zusammenhalt des Schwammes in Mitleidenschaft gezogen wird und er seine Integrität verliert. Im Zusammenspiel mit der Plasmabehandlung, durch die nicht nur die Benetzbarkeit der äußeren, sondern vorzugsweise auch der gesamten inneren Oberfläche der Zellstruktur verbessert wird, lässt sich auf diese Weise ein Gelatineschwamm herstellen, der bei Kontakt mit einem wässrigen Medium dieses rasch aufsaugt und vollständig benetzt und hydratisiert wird.

Das mechanische Komprimieren des Materials wird vor der Plasmabehandlung durchgeführt, da in diesem Fall die Plasmabehandlung in Bezug auf die innere Oberfläche der Zellstruktur effektiver ist.

Um einen merklichen Effekt durch die mechanische Kompression zu erzielen, wird der Gelatineschwamm bevorzugt in mindestens einer Richtung um ca. 40% oder mehr komprimiert, weiter bevorzugt um ca. 50% oder mehr. Das Komprimieren in nur einer Richtung, z.B. durch Walzen des Materials, ist häufig ausreichend, es ist jedoch auch ein Komprimieren des Materials in mehreren Richtungen möglich.

Bei der Benetzung und Hydratisierung eines komprimierten, plasmabehandelten Gelatineschwamms wird die Kompression zum Teil wieder rückgängig gemacht, d.h. der Schwamm expandiert beim Absorbieren des wässrigen Mediums. Dieser Effekt kann ausgenutzt werden, um Gelatineschwämme herzustellen, die bei Kontakt mit Flüssigkeiten nicht nur schnell benetzt werden, sondern auch in erheblichem Umfang ihr Volumen vergrößern, z.T. bis zu ihrer ursprünglichen Größe vor der Kompression. In diesem Fall ist es bevorzugt, wenn das Material im Zuge des erfindungsgemäßen Verfahrens in mindestens einer Richtung um ca. 65% oder mehr komprimiert wird, weiter bevorzugt um ca. 75% oder mehr.

Solche stark komprimierten, schnell benetzbaren Gelatineschwämme sind nicht nur in der Lage, besonders große Mengen an Blut oder auch anderen Körperflüssigkeiten aufzunehmen, sondern können auch eine Stütz- oder Verschlussfunktion für das umliegende Gewebe übernehmen. Insbesondere bei der postoperativen Wundversorgung bieten solche Gelatineschwämme hervorragende Vorraussetzungen im Vergleich zu nichtresorbierbaren Materialien, da aufgrund der Bioabbaubarkeit von Gelatine kein weiterer Eingriff zur Entfernung des Materials erforderlich ist.

Besonders vorteilhaft können schnell benetzbare Gelatineschwämme mit hohem Expansionsvermögen auch als Nasaltampons eingesetzt werden, und zwar sowohl zur Notfallversorgung bei starken Fällen von Nasenbluten als auch bei Operationen im Nasalbereich (insbesondere bei der funktionellen endoskopischen Sinuschirurgie sowie bei der Turbinoplastik). Bei den genannten Indikationen müssen relativ große Mengen Blut möglichst rasch aufgenommen werden.

Um eine möglichst gleichförmige Expansion des Gelatineschwammes zu gewährleisten, kann die Kompression in mehreren Richtungen erfolgen (zum Beispiel kann eine radiale Kompression bei einem zylindrischen Schwamm, insbesondere einem Nasaltampon, durchgeführt werden).

Ein weiterer chirurgischer Eingriff, bei dem es gelegentlich zu postoperativen Blutungen kommt, ist die Tonsillektomie (Entfernung der Gaumenmandeln). Auch in diesem Fall können solche Blutungen mit Hilfe von schnell benetzbaren Gelatineschwämmen versorgt werden.

Je nach Einsatzbereich können Gelatineschwämme mit einer höheren oder geringeren Härte im hydratisierten Zustand gewünscht sein. Möglichst weiche Materialien sind zum Beispiel unter dem Gesichtspunkt der Vermeidung von Narbenbildung im Falle der funktionellen endoskopischen Sinuschirurgie bevorzugt, während bei der Turbinoplastlk Gelatineschwämme mit einer etwas höheren Festigkeit vorteilhaft sind, um einen gewissen Druck auf die Nasenarterie auszuüben. Die Härte der Gelatineschwämme in hydratisiertem Zustand kann insbesondere durch die Dichte des Materials sowie die Zellstruktur beeinflusst werden.

Bei einer weiteren Ausführungsform der Erfindung umfasst das Gelatine enthaltende Material einen oder mehrer pharmazeutische Wirkstoffe, die je nach Anwendungsfall ausgewählt sein können. Zur weiteren Verbesserung der blutungsstillenden Wirkung können Gelatineschwämme insbesondere Hämostatika umfassen.

Die Gelatineschwämme können des Weiteren eingefärbt sein, um dem Anwender die Unterscheidung verschiedener Produkte zu erleichtern. Dies gilt gleichermaßen für andere Gelatine enthaltende Materialien, die im Folgenden beschrieben werden.

### Weitere Aspekte der Erfindung

Gegenstand der vorliegenden Erfindung ist ferner ein schnell benetzbares, natürliches Hydrokolloid enthaltendes Material in Gestalt eines Formkörpers, welches gemäß dem vorstehend beschriebenen Verfahren erhältlich ist. Besondere Vorteile und Ausführungsformen des erfindungsgemäßen Materials wurden bereits im Zusammenhang mit dem erfindungsgemäßen Verfahren erläutert.

Das erfindungsgemäße Material kann ferner über seine Anfangsbenetzbarkeit definiert werden, die sich in der Geschwindigkeit niederschlägt, mit der eine bestimmte Menge eines wässrigen Mediums von dem Material absorbiert wird.

Gemäß einer bevorzugten Ausführungsform betrifft die Erfindung ein schnell benetzbares, Hydrokolloid enthaltendes Material mit einer Zellstruktur, wobei das Material eine derartige Anfangsbenetzbarkeit aufweist, dass es 50 µl eines wässrigen Mediums innerhalb von maximal 10 Sekunden, insbesondere maximal 5 Sekunden, absorbiert. Das Hydrokolloid enthaltende Material ist insbesondere ein Gelatine enthaltendes Material in Form eines Gelatineschwammes.

Bevorzugt weist dieses Material ferner eine derartige Quellfähigkeit auf, dass es beim Eintauchen in Wasser innerhalb von maximal 6 Sekunden sein Volumen um mindestens 200% vergrößert. Ein solches Material kann insbesondere durch die vorherige Kompression des Materials mit der Zellstruktur erhalten werden.

Die Messung der Anfangsbenetzbarkeit und der Volumenzunahme sind in den nachfolgenden Beispielen im Einzelnen beschrieben.

Die Erfindung betrifft weiterhin die Verwendung eines erfindungsgemäßen Hydrokolloid enthaltenden Materials bei der Herstellung und/oder Verarbeitung von Lebensmitteln, wie oben ausgeführt.

Ferner betrifft die Erfindung die Verwendung der erfindungsgemäßen Materialien in der Human- oder Veterinärmedizin, insbesondere zur Förderung der Hämostase (d.h. zum Stillen von Blutungen), als Wundauflage und/oder als Nasaltampon, sowie als Substrat für lebende Zellen und/oder zur Herstellung von Gewebeimplantaten. Für Einzelheiten der jeweiligen Verwendungen wird ebenfalls auf die obige Beschreibung des erfindungsgemäßen Verfahrens verwiesen.

Diese und weitere Vorteile der Erfindung werden anhand der folgenden Beispiele unter Bezugnahme auf die Figuren näher erläutert. Es zeigen im Einzelnen:
- Figur 1:: schematische Darstellung eines Versuchs zur Bestimmung der Anfangsbenetzbarkeit;
- Figur 2:: schematische Darstellung eines Versuchs zur Bestimmung der Geschwindigkeit der Blutabsorption;
- Figur 3:: Diagramm betreffend die Geschwindigkeit der Blutabsorption durch plasmabehandelte (erfindungsgemäße) sowie nicht-plasmabehandelte Gelatineschwämme; und
- Figur 4:: fotografische Darstellung der Quellung eines stark komprimierten Gelatineschwammes.

### Beispiele

### Beispiel 1: Herstellung eines schnell benetzbaren Gelatineschwammes

Dieses Beispiel beschreibt den Einsatz des erfindungsgemäßen Verfahrens zur Herstellung eines schnell benetzbaren, Hydrokolloid enthaltenden Materials in Form eines Gelatineschwammes.

Als Ausgangsmaterial wurden handelsübliche Gelatineschwämme eingesetzt, die als Wundauflage verwendet werden. Durch Vernetzung der Gelatine sind diese Schwämme in Wasser unlöslich, aber unter physiologischen Bedingungen abbaubar.

Die einzelnen Gelatineschwämme, die ursprünglich Abmessungen von 80x50x10 mm aufwiesen, wurden in einem ersten Schritt auf eine Dicke von 4 mm gewalzt, sodass sich Abmessungen von 80x50x4 mm ergaben. Durch dieses Komprimieren der Gelatineschwämme in einer Richtung um 60% wurde die Zellstruktur teilweise aufgebrochen, um eine möglichst hohe Effizienz der nachfolgenden Plasmabehandlung in Bezug auf die gesamte innere Oberfläche der Schwämme zu ermöglichen.

Anschließend wurden die Schwämme im Vakuum (< 1 mbar) für 2 Stunden bei 50 °C getrocknet, um den Wassergehalt der Gelatine, der in der Regel bei ca. 8 bis ca. 12 Gew.-% liegt, möglichst weit zu reduzieren, z.B. auf 2-7 Gew.-%.

70 gewalzte und getrocknete Gelatineschwämme mit den Abmessungen 80x50x4 mm wurden mit einem O₂-Plasma in der Plasmakammer (Volumen 30 I) einer Niederdruckplasmaanlage mit Hochfrequenzeinspeisung bei 40 kHz für eine Dauer von 30 Minuten behandelt. Hierbei wurden die Maschinenparameter gemäß folgender Tabelle 1 eingestellt:

**Tabelle 1**

| **Parameter** | **Sollwert** |
|---|---|
| | |
| Abpumpdruck | 0,4 mbar |
| Maximale Abpumpdauer | 10 min |
| Gasflussanteil | 100 % O₂ |
| Maximal zulässige Gasabweichung | 10 % |
| Stabilisierungszeit | 1 min |
| Prozessdruck | 0,5 mbar |
| Maximal zulässige Druckabweichung | +/- 0,25 mbar |
| Leistung | 1440 W |
| Maximal zulässige Leistungsabweichung | 15 % |
| Prozessdauer | 30 min |
| Spülzeit | 1 min |
| Belüftungszeit | 1 min |

Die Gelatineschwämme wurden während der Plasmabehandlung so gelagert, dass sie von allen sechs Seiten her gleichmäßig dem Plasma ausgesetzt waren.

Erhalten wurden rein weiße Schwämme mit niedrigen Benetzungszeiten.

### Beispiel 2: Bestimmung der Anfangsbenetzbarkeit

Zur quantitativen Bestimmung der Benetzbarkeit der erfindungsgemäßen, plasmabehandelten Gelatineschwämme wurde die benötigte Zeit für die Absorption einer bestimmten Menge einer wässrigen Lösung durch den trockenen Schwamm gemessen (Anfangsbenetzbarkeit).

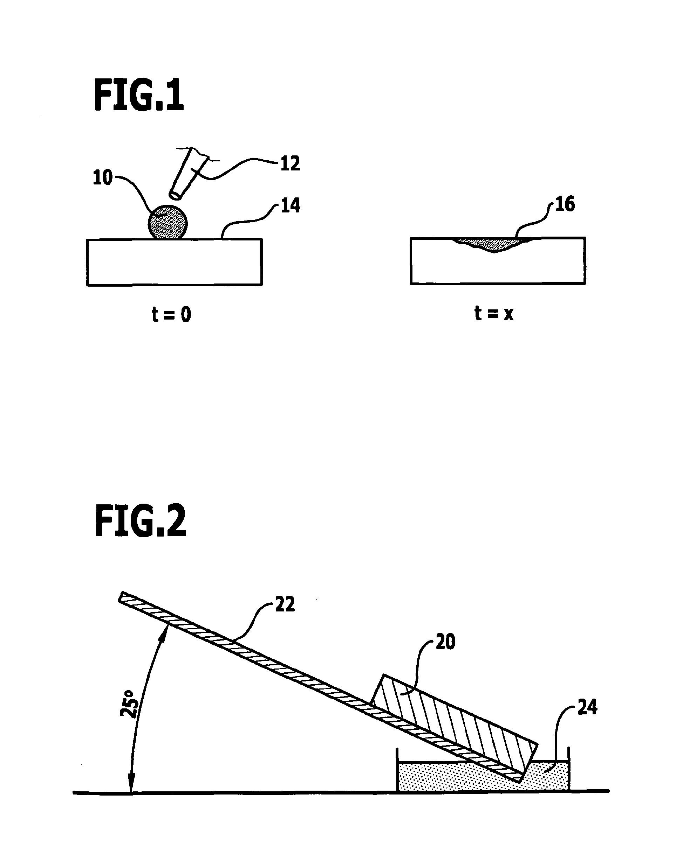

Die Durchführung des Versuchs ist in der Figur 1 schematisch dargestellt. Es wurden jeweils 50 µl eines PBS-Puffers mit 0,03 Gew.-% Methylenblau (10) mit einer Pipette (12) auf eine Stelle der Schwammoberfläche (14) gegeben. Gemessen wurde die Zeitspanne von der Aufgabe des Tropfens (t=0) bis zu dessen vollständigen Eindringen in den Schwamm (t=x). Der Endpunkt wurde visuell bestimmt durch das Erkennen der Schwammstruktur unterhalb des einsinkenden Tropfens (16).

Es wurden jeweils mindestens drei Gelatineschwämme untersucht und die Messung wurde bei jedem Schwamm an vier bis sechs Stellen der Oberfläche durchgeführt. Aus allen diesen Messungen wurde ein Mittelwert gebildet.

Für die gemäß Beispiel 1 hergestellten Gelatineschwämme ergab sich unmittelbar nach der Plasmabehandlung ein durchschnittlicher Wert für die Anfangsbenetzbarkeit von 4,4 Sekunden mit einer Standardabweichung von 1,7 Sekunden.

Die Messung wurde wiederholt, nachdem die plasmabehandelten Schwämme 24 Stunden unter Atmosphärenbedingungen gelagert wurden. Dabei nahmen die vor der Plasmabehandlung getrockneten Schwämme wieder eine gewisse Menge an Feuchtigkeit auf. In diesem Fall wurde eine Anfangsbenetzbarkeit von durchschnittlich 3,6 Sekunden mit einer Standardabweichung von 0,9 Sekunden gemessen.

Im Gegensatz hierzu war die Anfangsbenetzbarkeit der in Beispiel 1 als Ausgangsmaterial dienenden, unbehandelten Gelatineschwämme so gering, dass der auf die Oberfläche aufgesetzte Tropfen PBS-Puffer selbst nach 20 Minuten noch nicht in den Schwamm eingedrungen war.

Dieses Beispiel zeigt eindrucksvoll, dass die Benetzbarkeit von Gelatineschwämmen durch die erfindungsgemäße Plasmabehandlung deutlich verbessert wird. Dies ist für die medizinische Anwendung solcher Schwämme von erheblichem Vorteil, wie auch in den folgenden Beispielen gezeigt wird.

### Beispiel 3: Bestimmung der Blutabsorptionskapazität

In diesem Beispiel wurde die Blutabsorptionskapazität eines erfindungsgemäßen Gelatineschwammes (hergestellt gemäß Beispiel 1) im Vergleich zu dem nicht-plasmabehandelten Ausgangsprodukt quantitativ bestimmt.

Hierfür wurden die trockenen Schwämme jeweils gewogen und dann für 20 Sekunden auf die Oberfläche einer Blutprobe (humanes Blut mit einem Hämatokrit von ca. 50%) gelegt. Anschließend wurden die Schwämme für 60 Sekunden auf ein Filterpapier gelegt, um überschüssiges Blut abfließen zu lassen. Die Schwämme mit dem absorbierten Blut wurden erneut gewogen und anhand der Differenz zum Ursprungsgewicht wurde die Menge an absorbiertem Blut bezogen auf das Gewicht des Schwammmaterials berechnet. Es wurde jeweils der Mittelwert aus drei Messungen gebildet.

Auch bei diesem Versuch zeigte sich ein deutlicher Unterschied zwischen den beiden Schwämmen: Während der plasmabehandelte Gelatineschwamm das ca. 58-fache seines Eigengewichts an Blut aufnahm, lag der entsprechende Wert bei dem trockenen, unbehandelten Gelatineschwamm bei ca. dem 1,0-fachen des Eigengewichts, was für die praktische Anwendung nahezu vernachlässigbar ist.

Die schnelle Benetzbarkeit des erfindungsgemäßen Gelatineschwammes führt somit zu einer wesentlich höheren Blutabsorptionskapazität beim Inkontaktbringen des trockenen Schwammes mit Blut.

In einem weiteren Versuch zur Blutabsorptionskapazität wurde die Geschwindigkeit bestimmt, mit der das Blut (gegen die Schwerkraft) von dem Gelatineschwamm aufgesaugt wird.

Es wurden auch hier der unbehandelte Schwamm sowie der plasmabehandelte Schwamm gemäß Beispiel 1 verwendet, und zwar sowohl trocken als auch in einem angefeuchteten bzw. benetzten Zustand. Zum Anfeuchten der Schwämme wurden diese vor der Durchführung des Versuchs vollständig in Wasser eingetaucht und anschließend ausgedrückt.

Die Durchführung des Versuchs ist in der Figur 2 schematisch dargestellt.

Die untersuchten Schwämme (20) wiesen jeweils eine Länge von 80 mm, eine Breite von 20 mm und eine Dicke von 10 bzw. 4 mm auf (siehe Beispiel 1). Die Schwämme (trocken bzw. angefeuchtet) wurden auf ein Lineal (22) gelegt, sodass die Skalierung des Lineals entlang der 80 mm langen Seite des Schwammes verlief. Das Lineal mit dem Schwamm wurde in einem Winkel von 25° zur Waagerechten gehalten und das untere Ende des Schwammes (d.h. die 20 mm breite Seite) in ein mit 0,9 Gew.-%iger Kochsalzlösung verdünntes Blutkonzentrat (Hämatokrit von ca. 50%) eingetaucht (24). Beginnend mit dem Zeitpunkt des Eintauchens wurde in Abhängigkeit von der Zeit die Steighöhe des Blutes in dem Gelatineschwamm gemessen, d.h. es wurde die Strecke anhand der Linealskala abgelesen, die das Blut durch Absorption in dem Schwamm zurückgelegt hatte.

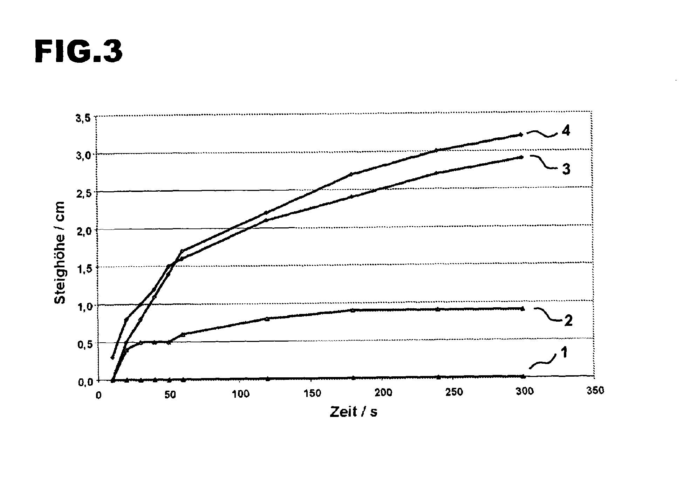

Das Ergebnis dieses Versuches ist in der Figur 3 dargestellt. Diese zeigt ein Diagramm, in der die Steighöhe des Blutes in cm in Abhängigkeit von der Zeit in Sekunden für die vier untersuchten Gelatineschwämme dargestellt ist.

Bei dem trockenen, nicht plasmabehandelten Schwamm (Kurve 1) erfolgte während des gesamten Beobachtungszeitraums von 5 Minuten keine erkennbare Absorption des Blutes in den Schwamm. Wurde dieser Schwamm zuvor angefeuchtet (Kurve 2), erfolgte während der ersten drei Minuten eine Blutabsorption bis zu einer Steighöhe von knapp 1 cm, die dann konstant blieb. Dies zeigt, dass der nicht-plasmabehandelte Gelatineschwamm aufgrund seiner schlechten Benetzbarkeit zuvor angefeuchtet werden muss, um überhaupt eine Absorptionswirkung zu zeigen.

Demgegenüber zeigt der mit dem erfindungsgemäßen Verfahren plasmabehandelte Gelatineschwamm sowohl in feuchtem Zustand (Kurve 3) als auch in trockenem Zustand (Kurve 4) eine deutlich schnellere Absorption des Blutes, d.h. eine höhere Saugfähigkeit. In beiden Fällen stieg das Blut während des Beobachtungszeitraumes kontinuierlich bis auf eine Höhe von ca. 3 cm, d.h. auf die mehr als 3-fache Höhe im Vergleich zu dem feuchten, nicht-plasmabehandelten Schwamm.

Während der Gelatineschwamm gemäß dem Stand der Technik zuvor angefeuchtet werden muss, um überhaupt zur Absorption von Blut (d.h. z.B. als Wundauflage) eingesetzt werden zu können, ist dies bei dem erfindungsgemäßen Gelatineschwamm nicht erforderlich, da dieser bereits in trockenem Zustand eine deutlich höhere Absorptionswirkung bzw. Saugkraft aufweist.

### Beispiel 4: Untersuchung der hämostatischen Wirkung

Die hämostatische (blutungsstillende) Wirkung des plasmabehandelten Gelatineschwammes gemäß Beispiel 1 sowie des nicht-plasmabehandelten Vergleichsmaterials (GELITA-SPON) wurde mit Hilfe eines validierten *ex vivo*-Venenmodells unter standardisierten Bedingungen untersucht.

Dabei wurde ein mittels Varektomie gewonnener, in sich abgeschlossener Abschnitt der Vena saphena magna mit Blut befüllt und mittels definierter Punktion eine diffuse Blutung hervorgerufen. Der Blutdruck in der Vene wurde während des gesamten Versuches konstant bei 30 mm Hg gehalten. Nach einer Blutungsdauer von 5 Sekunden wurde die Hämostase durch Aufbringen des jeweiligen Gelatineschwammes auf die Punktion eingeleitet.

Es wurde sowohl die Zeit bis zur vollständigen Stillung der Blutung als auch der Blutverlust erfasst.

Als Ergebnis mehrerer Versuche ergab sich für den nicht-plasmabehandelten Gelatineschwamm eine durchschnittliche Zeit bis zur vollständigen Hämostase von ca. 5,5 Minuten, bei einem durchschnittlichen Blutverlust von ca. 8 ml. Demgegenüber führte die erfindungsgemäße Plasmabehandlung des Gelatineschwammes zu einer erheblichen Verkürzung der durchschnittlichen Hämostasezeit auf ca. 3 Minuten und zu einer Halbierung des durchschnittlichen Blutverlustes auf ca. 4 ml.

Der Versuch zeigt, dass die hämostatische Wirkung von Gelatineschwämmen durch die erfindungsgemäße Plasmabehandlung überraschenderweise deutlich verbessert werden kann.

### Beispiel 5: Herstellung von schnell benetzbaren, stark komprimierten Gelatineschwämmen

Die in diesem Beispiel untersuchten Gelatineschwämme wurden auf folgende Weise hergestellt:
Eine 15 Gew.-%ige Lösung von Schweineschwartengelatine (300 g Bloom) wurde hergestellt, indem die Gelatine zunächst in Wasser gequollen und dann bei 60 °C gelöst wurde. Die Lösung wurde mittels Ultraschall entgast und mit 1 molarer Natronlauge auf einen pH-Wert von 7,2 eingestellt. Anschließend wurde eine entsprechende Menge einer wässrigen 1 Gew.-%igen Formaldehydlösung als Vernetzungsmittel zugegeben, sodass 2.000 ppm Formaldehyd (bezogen auf die Gelatine) vorlagen.

Die Lösung wurde nach einer Reaktionszeit von 5 Minuten auf ca. 45 °C temperiert und maschinell mit Luft aufgeschäumt. Die aufgeschäumte Gelatinelösung, die eine Nassdichte von 120 g/l aufwies, wurde in eine Form mit einer Abmessung von 40x20x6 cm gegossen und bei einer relativen Luftfeuchtigkeit von 10% zunächst für 2 Stunden bei 20 °C und anschließend für 5 bis 7 Tage bei 26 °C getrocknet.

Aus dem getrockneten Gelatineschwamm wurden drei verschiedene Proben mit folgenden Abmessungen ausgeschnitten (Abmessungen axbxc):

| | |
|---|---|
| Probe 1: | 40x150x35 mm |
| Probe 2: | 50x150x35 mm |
| Probe 3: | 80x150x35 mm |

Alle drei Proben wurden anschließend einer starken Kompression entlang einer Raumrichtung unterzogen, wobei die Abmessung c (zuvor 35 mm) durch Walzen auf 10 mm im Falle der Proben 1 und 3 und auf 15 mm im Falle der Probe 2 reduziert wurde. In der Abmessung b (zuvor 150 mm) wurden die Schwämme anschließend nochmals zugeschnitten, sodass sich Proben mit folgenden Abmessungen (Sollwerte) ergaben:

| | |
|---|---|
| Probe 1: | 40x7x10 mm |
| Probe 2: | 50x5x15 mm |
| Probe 3: | 80x10x10 mm |

Die Behandlung der drei verschiedenen Schwammproben mit einem O₂-Niederdruckplasma erfolgte wie in Beispiel 1 beschrieben, abweichend von den dort angegebenen Maschinenparametern jedoch mit einem Abpumpdruck von 0,15 mbar und einem Prozessdruck von 0,2 mbar.

### Beispiel 6: Benetzungs- und Expansionsverhalten der stark komprimierten Gelatineschwämme

Die gemäß Beispiel 5 hergestellten, komprimierten und plasmabehandelten Gelatineschwämme wurden in Wasser eingetaucht, was zu einer raschen Benetzung und Quellung der Schwämme führte. Dabei wurden die Quelldauer, die Abmessungen der Schwämme vor und nach dem Quellen sowie die Volumenzunahme gemessen. Von jeder Probe wurden zwei Exemplare untersucht. Die Ergebnisse sind in der nachfolgenden Tabelle 2 dargestellt:

**Tabelle 2**

| | Quelldauer | Maße vor dem Quellen (mm) | Maße nach dem Quellen (mm) | Volumenzunahme |
|---|---|---|---|---|
| Probe 1-1 | 5 Sekunden | 40x7x11 | 42x6x40 | 227% |
| 1-2 | 4 Sekunden | 40x7x11 | 42x7x40 | 282% |
| Probe 2-1 | 6 Sekunden | 50x5x16 | 53x5x39 | 158% |
| 2-2 | 6 Sekunden | 50x5x16 | 53x4x40 | 112% |
| Probe 3-1 | 4 Sekunden | 79x9x11 | 82x9x39 | 268% |
| 3-2 | 5 Sekunden | 79x9x12 | 83x10x42 | 309% |

Diese Ergebnisse zeigen, dass die plasmabehandelten Gelatineschwämme bei Flüssigkeitskontakt nicht nur sehr schnell benetzen, sondern auch in erheblichem Umfang aufquellen und ihr Volumen expandieren. Bei entsprechender Kompression der Gelatineschwämme können Volumenzunahmen von deutlich über 200%, z.T. sogar über 300%, erzielt werden.

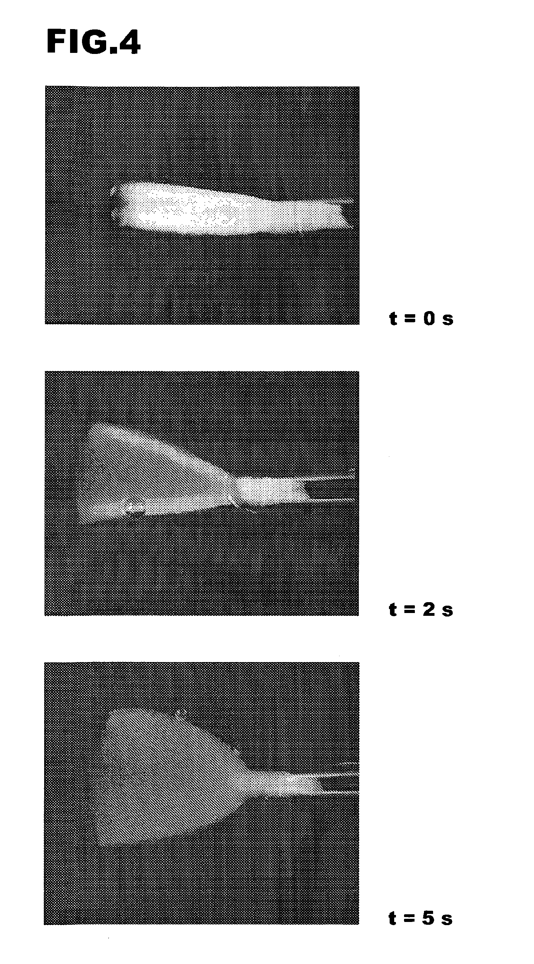

Die Quellung eines stark komprimierten Gelatineschwammes beim Eintauchen in Wasser ist in der Figur 4 fotografisch dargestellt. Die einzelnen Abbildungen zeigen den Schwamm unmittelbar vor dem Eintauchen (t=0 s), sowie 2 Sekunden bzw. 5 Sekunden nach dem Eintauchen. Der Schwamm wird dabei an der rechten Seite von einer Pinzette gehalten, was ein Aufquellen in diesem Bereich verhindert. Im linken Bereich des Schwammes zeigt sich hingegen deutlich die erhebliche Volumenzunahme innerhalb kurzer Zeit.

Solche stark expandierenden Gelatineschwämme sind z.B. für die Verwendung als Nasaltampon besonders geeignet.

### Beispiel 7: Herstellung von schnell benetzbaren Gelatinevliesen

Faserwirrgelege aus Gelatinefasern (Gelatinevliese) wurden gemäß dem in der deutschen Patentanmeldung Nr. 10 2007 011 606 beschriebenen Rotationsspinnverfahren hergestellt.

Ausgangspunkt für die Herstellung war eine 20 Gew.-%ige wässrige Lösung von Schweineschwartengelatine (300 g Bloom), die mittels einer Rotationsspinnvorrichtung, wie sie z.B. auch in der DE 10 2005 048 939 A1 beschrieben ist, zu einem Wirrgelege aus Gelatinefasern versponnen wurde. Mehrere Lagen des Wirrgeleges wurden zu einem Gelatinevlies mit einer Dicke von ca. 1,7 mm flächig aufeinander gelegt.

Die Gelatine in dem Vlies wurde anschließend vernetzt, indem dieses für 17 Stunden dem Gleichgewichtsdampfdruck einer 10 Gew.-%igen Formaldehydlösung ausgesetzt wurde. Anschließend wurde das Vlies 48 Stunden bei ca. 50 °C und ca. 70% relativer Luftfeuchtigkeit getempert, um die Vernetzungsreaktion zu vervollständigen und den überschüssigen Anteil an nicht verbrauchtem Formaldehyd zu entfernen.

Aus dem Gelatinevlies wurden Stücke mit einer Abmessung von 20x20 mm ausgeschnitten und einer erfindungsgemäßen Plasmabehandlung unterworfen. Dies erfolgte wie in Beispiel 1 beschrieben, abweichend von den dort angegebenen Maschinenparametern jedoch bei einem Prozessdruck von 0,2 mbar und einer Prozessdauer von 15 Minuten.

Beim Inkontaktbringen des trockenen, plasmabehandelten Gelatinevlieses mit humanem Blut erfolgte eine schlagartige Benetzung und Absorption des Blutes durch das Vlies. Dabei wurde das plasmabehandelte Vlies innerhalb von weniger als 1 Sekunde vollständig durchtränkt, während ein unbehandeltes Vlies mit gleichen Abmessungen erst nach 45 Sekunden durchtränkt wurde. Gelatinevliese können in ähnlicher Weise wie die oben beschriebenen Gelatineschwämme zur Förderung der Hämostase und/oder zur Wundversorgung im medizinischen Bereich eingesetzt werden.

## Patentansprüche

1. Verfahren zur Herstellung eines schnell benetzbaren Formkörpers aus einem Gelatine enthaltenden Material, bei dem ein Formkörper aus einem Gelatine enthaltenden Material mit einer Zellstruktur zuerst mechanisch komprimiert und anschließend einem Plasma ausgesetzt wird.

2. Verfahren nach Anspruch 1, wobei das Material vollständig aus Gelatine besteht.

3. Verfahren nach einem der vorangehenden Ansprüche, wobei das Gelatine enthaltende Material getrocknet wird, bevor es dem Plasma ausgesetzt wird.

4. Verfahren nach einem der vorangehenden Ansprüche, wobei das Plasma ein Niederdruckplasma ist.

5. Verfahren nach einem der vorangehenden Ansprüche, wobei das Material dem Plasma länger als 1 Minute ausgesetzt wird.

6. Verfahren nach einem der vorangehenden Ansprüche, wobei die Zellstruktur einen mittleren Porendurchmesser von weniger als 300 µm aufweist.

7. Verfahren nach einem der vorangehenden Ansprüche, wobei der Formkörper in mindestens einer Richtung um 40% oder mehr komprimiert wird.

8. Verfahren nach einem der vorangehenden Ansprüche, wobei das Material einen oder mehrere pharmazeutische Wirkstoffe umfasst.

## Claims

1. A method for producing a quickly wettable shaped body made from a gelatin containing material, in which a shaped body made from a gelatin containing material with a cellular structure is first mechanically compressed and is then exposed to a plasma.

2. The method according to claim 1, wherein the material consists completely of gelatin.

3. The method according to one of the preceding claims, wherein the gelatin containing material is dried before it is exposed to the plasma.

4. The method according to one of the preceding claims, wherein the plasma is a low-pressure plasma.

5. The method according to one of the preceding claims, wherein the material is exposed to the plasma for longer than 1 minute.

6. The method according to one of the preceding claims, wherein the cellular structure has an average pore diameter of less than 300 µm.

7. The method according to one of the preceding claims, wherein the material is compressed in at least one direction by 40% or more.

8. Method according to one of the preceding claims, wherein the material comprises one or more pharmaceutical active substances.

## Revendications

1. Procédé de fabrication d'un corps moulé rapidement mouillable constitué d'une matière contenant de la gélatine, dans lequel un corps moulé constitué d'une matière contenant de la gélatine ayant une structure cellulaire est d'abord comprimé mécaniquement et ensuite soumis à un plasma.

2. Procédé selon la revendication 1, dans lequel la matière est entièrement constituée de gélatine.

3. Procédé selon l'une des revendications précédentes, dans lequel la matière contenant de la gélatine est séchée avant de la soumettre au plasma.

4. Procédé selon l'une des revendications précédentes, dans lequel le plasma est un plasma basse pression.

5. Procédé selon l'une des revendications précédentes, dans lequel la matière est soumise au plasma pendant plus de 1 minute.

6. Procédé selon l'une des revendications précédentes, dans lequel la structure cellulaire présente un diamètre moyen de pores inférieur à 300 µm.

7. Procédé selon l'une des revendications précédentes, dans lequel le corps moulé est comprimé à 40 % ou plus dans au moins une direction.

8. Procédé selon l'une des revendications précédentes, dans lequel la matière comprend une ou plusieurs substances actives pharmaceutiques.
